# EUROPEAN PATENT SPECIFICATION

(11) **EP 3 331 387 B1**
(45) Date of publication and mention of the grant of the patent: **19.02.2020**
(21) Application number: 16745678.9
(22) Date of filing: 25.07.2016
(51) Int. Cl.: A24F 47/00

(54) **AN AEROSOL-GENERATING SYSTEM WITH ENHANCED AIRFLOW MANAGEMENT**
AEROSOLERZEUGUNGSSYSTEM MIT VERBESSERTEM LUFTSTROMMANAGEMENT
SYSTÈME DE GÉNÉRATION D'AÉROSOL AVEC GESTION AMÉLIORÉE DE L'ÉCOULEMENT D'AIR

(30) Priority: 07.08.2015 EP 15180209
(43) Date of publication of application: 13.06.2018
(73) Proprietor: Philip Morris Products S.A., 2000 Neuchâtel (CH)
(72) Inventor: THORENS, Michel, 1510 Moudon (CH)
(74) Representative: Ponder, William Anthony John
(86) International application number: PCT/EP2016/067702
(87) International publication number: WO 2017/025310

(56) References cited:
- CN-U- 203 986 096
- US-A1- 2014 190 478
- US-A1- 2015 090 279
- US-A1- 2015 101 606
- US-A1- 2015 114 409

## Description

The present invention relates to aerosol-generating systems that comprise a heater assembly suitable for vaporising a liquid soaked from a capillary medium. In particular, the invention relates to handheld aerosol-generating systems, such as electrically operated smoking systems.

One type of aerosol-generating system is an electrically operated smoking system. Handheld electrically operated smoking systems consisting of a device portion comprising a battery and control electronics, a cartridge portion comprising a supply of aerosol-forming substrate, and an electrically operated vaporiser, are known. A cartridge comprising both a supply of aerosol-forming substrate and a vaporiser is sometimes referred to as a "cartomiser". The vaporiser typically comprises a coil of heater wire wound around an elongate wick soaked in liquid aerosol-forming substrate. The cartridge portion typically comprises not only the supply of aerosol-forming substrate and an electrically operated vaporiser, but also a mouthpiece, which the user sucks on in use to draw aerosol into their mouth.

US 2015/0090279 discloses an electronic cigarette comprising a liquid reservoir comprising a heat resistant sponge. Liquid is absorbed in the liquid reservoir. A foam metal heating plate is bonded to the bottom wall of the liquid reservoir. An air passage is formed through both the reservoir and the heating plate.

CN203986096 A discloses an electronic cigarette having an atomizer. The atomizer comprises a spiral electric heating element, coiled in a disk shape. The heating element is laid on a tar guiding cloth that covers an opening of a tar storage space. An opening is formed through the heating element, tar guiding cloth and tar storage space for the passage of air.

The present invention is directed to an aerosol-generating system which offers improved aerosolization and better aerosol droplet growth and which avoids occurrence of hot spots especially in the middle part of the heater assembly.

It would be desirable to provide an aerosol-generating system that improves the airflow on the surface of the heater assembly to encourage the mixing of the volatized vapors. It would be further desirable to provide an aerosol-generating system that accelerates the airflow of the aerosol from the heater assembly towards the mouthpiece, thereby further improving the aerosolization through faster cooling of the volatized vapors. In embodiments, enhanced mixing and acceleration of airflow is achieved by the introduction of turbulence and vortices.

According to the invention there is provided an aerosol-generating system

comprising a liquid storage portion comprising a housing holding a liquid aerosol-forming substrate and a capillary medium, the housing having an opening. A fluid permeable heater assembly comprising an arrangement of electrically conductive filament is arranged to define an air impingement surface, wherein the fluid permeable heater assembly extends across the opening of the housing, and wherein the filaments arrangement defines a filament opening allowing airflow to pass through. The capillary medium is provided in contact with the heater assembly. The liquid aerosol-forming substrate is drawn via the capillary medium to the electrically conductive filament arrangement. The capillary medium comprises a capillary medium opening extending the filament opening through the capillary medium. The aerosol-generating system comprises a main housing and an air inlet provided in a sidewall of the main housing, such that ambient air may be drawn towards the heating element from the air inlet at an angle of approximately or up to 90° with respect to an air duct defined by the capillary medium opening.

The present invention is further directed to a method of manufacture of a cartridge for use in an electrically operated aerosol-generating system, comprising the stpes of providing a liquid storage portion comprising a housing having an opening, providing a capillary material within the liquid storage portion, filling the liquid storage portion with liquid aerosol-forming substrate and providing a fluid permeable heater assembly comprising an arrangement of electrically conductive filaments arranged to define an air impingement surface, wherein the fluid permeable heater assembly extends across the opening of the housing, and wherein the filament arrangement defines a filament opening allowing airflow to pass through. The capillary medium is provided in contact with the heater assembly and the capillary medium comprises a capillary medium opening allowing airflow to pass through the capillary medium.

The provision of a heater assembly that extends across an opening of a liquid storage portion allows for a robust construction that is relatively simple to manufacture. This arrangement allows for a large contact area between the heater assembly and liquid aerosol-forming substrate. The housing may be a rigid housing. As used herein "rigid housing" means a housing that is self-supporting. The rigid housing of the liquid storage portion preferably provides mechanical support to the heater assembly.

The heater assembly may be substantially flat allowing for simple manufacture. As used herein, "substantially flat" means formed initially in a single plane and not wrapped around or other conformed to fit a curved or other non-planar shape. Geometrically, the term "substantially flat" electrically conductive filament arrangement is used to refer to an electrically conductive filament arrangement that is in the form of a substantially two dimensional topological contour or profile. Thus, the substantially flat electrically conductive filament arrangement extends in two dimensions along a surface substantially more than in a third dimension. In particular, the dimensions of the substantially flat filament arrangement in the two dimensions within the surface is at least 5 times larger than in the third dimension, normal to the surface. An example of a substantially flat filament arrangement is a structure between two substantially imaginary parallel surfaces, wherein the distance between these two imaginary surfaces is substantially smaller than the extension within the surfaces.

The term "filament" is used throughout the specification to refer to an electrical path arranged between two electrical contacts. A filament may arbitrarily branch off and diverge into several paths or filaments, respectively, or may converge from several electrical paths into one path. A filament may have a round, square, flat or any other form of cross-section. A filament may be arranged in a straight or curved manner.

The phrases "filament arrangement" or "arrangement of filaments" are used interchangeably throughout the specification to refer to an arrangement of a plurality of filaments. The filament arrangement may be an array of filaments, for example arranged parallel to each other. The filaments may form a mesh. The mesh may be woven or nonwoven. Throughout the specification, the surface of the filament arrangement that is in contact with the air flow is also referred to as "air impingement surface" of the filament arrangement.

The electrically conductive filaments may define interstices between the filaments and the interstices may have a width of between 10 micrometer and 100 micrometer. Preferably, the filaments give rise to capillary action in the interstices, so that in use, liquid to be vaporised is drawn into the interstices, increasing the contact area between the heater assembly and the liquid.

By providing the filament arrangement with a plurality of interstices for allowing fluid to pass through the filament arrangement, the filament arrangement is fluid permeable. This means that the aerosol-forming substrate, in a gaseous phase and possibly in a liquid phase, can readily pass through the filament arrangement and, thus, the heater assembly.

The substantially flat filament arrangement is configured for customizing the airflow around the air impingement surface. This is done by introducing turbulences and vortexes which encourage the mixing of volatized vapors and leading to enhanced aerosolization.

In some embodiments of the invention, the filament arrangement may be of planar shape, defining a planar air impingement surface.

In some embodiments of the invention, an initially substantially flat arrangement of filaments is deformed, shaped or otherwise modified to define an arrangement of filaments which define a non-planar air impingement surface. In an embodiment, an initially substantially flat filament arrangement is formed so that it is curved along one or more dimensions, for example forming a convex or "dome" shape, a concave shape, a bridge shape, or a cyclone or "funnel" shape. In an embodiment, the filament arrangement defines a concave surface which faces the airflow that arrives at and impinges upon the filament arrangement. The non-planar-shape of the filament arrangement encourages the introduction of turbulences and vortexes onto the airflow arriving at the filament arrangement. Position and shape of the filament arrangement are arranged such that an airflow guided to the air impingement surface of the filament arrangement is whirled around the air impingement surface.

The filament arrangement defines a filament opening allowing airflow to pass through. The capillary medium opening may extend the filament opening to form an air duct through the capillary medium. Position and shape of the filament arrangement, of the filament opening, and of the capillary medium opening are dimensioned and arranged such that an airflow guided to the air impingement surface of the filament arrangement is whirled around the air impingement surface.

The filament opening is substantially larger than the interstices between the filaments of the filament arrangement. Substantially larger means that the filament opening covers an area that is at least 5 times larger, or at least 10 times larger, or at least 50 times larger, or at least 100 times larger than the area of an interstice between two filaments. The relation of the area of the filament opening and the cross-section area of the filament arrangement including the filament opening may be at least 1 percent, or at least 2 percent, or at least 3 percent, or at least 4 percent, or at least 5 percent, or at least 10 percent, or at least 25 percent.

The position of the filament opening substantially may match the position of the capillary medium opening. Shape and size of the cross section of the filament opening may be the shape and size of the cross section of the capillary medium opening.

The heater assembly and the capillary medium may be arranged in an aerosol-generating system in such way that at least a portion of the airflow that arrives at the air impingement surface of the filament arrangement is guided through an air duct defined by the capillary medium opening through the capillary medium. The airflow through the air duct is accelerated by the suction of the air duct, thereby improving aerosolization through faster cooling of the volatized vapors.

Alternatively, the heater assembly and the capillary medium may be arranged such in an aerosol-generating system that the airflow arriving at the air impingement surface of the filament arrangement is guided through the air duct defined by the capillary medium opening through the capillary medium.

The electrically conductive filaments may form a mesh of size between 160 and 600 Mesh US (+/- 10 percent) (i.e. between 160 and 600 filaments per inch (+/- 10 percent)). The width of the interstices is preferably between 75 micrometer and 25 micrometer. The percentage of open area of the mesh, which is the ratio of the area of the interstices to the total area of the mesh is preferably between 25 percent and 56 percent. The mesh may be formed using different types of weave or lattice structures. Alternatively, the electrically conductive filaments consist of an array of filaments arranged parallel to one another. The mesh, array or fabric of electrically conductive filaments may also be characterised by its ability to retain liquid, as is well understood in the art.

The electrically conductive filaments may have a diameter of between 10 micrometer and 100 micrometer, preferably between 8 micrometer and 50 micrometer, and more preferably between 8 micrometer and 39 micrometer. The filaments may have a round cross section or may have a flattened cross-section.

The area of the mesh, array or fabric of electrically conductive filaments may be small, preferably less than or equal to 25 square millimeter, allowing it to be incorporated in to a handheld system. The mesh, array or fabric of electrically conductive filaments may, for example, be circular with a diameter of 3 millimeter to 10 millimeter, preferably 5 millimeter. The mesh may also be rectangular and, for example, have dimensions of 5 millimeter by 2 millimeter. Preferably, the mesh or array of electrically conductive filaments covers an area of between 10 percent and 50 percent of the area of the heater assembly. More preferably, the mesh or array of electrically conductive filaments covers an area of between 15 percent and 25 percent of the area of the heater assembly. Sizing of the mesh, array or fabric of electrically conductive filaments 10 percent and 50 percent of the area, or less or equal than 25 millimeter², reduces the amount of total power required to heat the mesh, array or fabric of electrically conductive filaments while still ensuring sufficient contact of the mesh, array or fabric of electrically conductive filaments to the liquid provided one or more capillary mediums to be volatilized.

The heater filaments may be formed by etching a sheet material, such as a foil. This may be particularly advantageous when the heater assembly comprises an array of parallel filaments. If the heater assembly comprises a mesh or fabric of filaments, the filaments may be individually formed and knitted together. Alternatively, the heaterfilaments may be stamped from electrically conductive foil, as for example stainless steel.

The filaments of the heater assembly may be formed from any material with suitable electrical properties. Suitable materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, constantan, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal®, iron-aluminium based alloys and iron-manganese-aluminium based alloys. Timetal® is a registered trade mark of Titanium Metals Corporation. The filaments may be coated with one or more insulators. Preferred materials for the electrically conductive filaments are 304, 316, 304L, 316L stainless steel, and graphite. Additionally, the electrically conductive filament arrangement may comprise combinations of the above materials. A combination of materials may be used to improve the control of the resistance of the substantially flat filament arrangement. For example, materials with a high intrinsic resistance may be combined with materials with a low intrinsic resistance. This may be advantageous if one of the materials is more beneficial from other perspectives, for example price, machinability or other physical and chemical parameters. Advantageously, a substantially flat filament arrangement with increased resistance reduces parasitic losses. Advantageously, high resistivity heaters allow more efficient use of battery energy. The battery energy is proportionally divided between the energy lost on the printed circuit board and the contacts and energy delivered to the electrically conductive filament arrangement. Thus the energy available for the electrically conductive filament arrangement in the heater is higher the higher the resistance of the electrically conductive filament arrangement.

Alternatively, the electrically conductive filament arrangement may be formed of carbon thread textile. Carbon thread textile has the advantage that it is typically more cost efficient than metallic heaters with high resistivity. Further, a carbon thread textile is typically more flexible than a metallic mesh. Another advantage is that the contact between a carbon thread textile and a transport medium like a high release material can be well preserved during construction of the fluid permeable heater assembly.

A reliable contact between the fluid permeable heater assembly and a transport medium, like for example a capillary transport medium such as a wick made from fibres or a porous ceramic material, improves the constant wetting of the fluid permeable heater assembly. This advantageously reduces the risk of overheating of the electrically conductive filament arrangement and inadvertent thermal decomposition of the liquid.

The heater assembly may comprise an electrically insulating substrate on which the filaments are supported. The electrically insulating substrate may comprise any suitable material, and is preferably a material that is able to tolerate high temperatures (in excess of 300 degrees Celsius) and rapid temperature changes. An example of a suitable material is a polyimide film, such as Kapton®. The electrically insulating substrate may have an aperture formed in it, with the electrically conductive filaments extending across the aperture. The heater assembly may comprise electrical contacts connected to the electrically conductive filaments. For example, the electrical contacts may be glued, welded or mechanically clamped to the electrically conductive filament arrangement. Alternatively the electrically conductive filament arrangement may be printed on the electrically insulating substrate, for example using metallic inks. In such an arrangement, preferably, the electrically insulating substrate is a porous material, such that the electrically conductive filament arrangement can be directly applied to the surface of the porous material. Preferably, in such an embodiment the porosity of the substrate functions as the "opening" of the electrically insulating substrate through which a liquid may be drawn towards the electrically conductive filament arrangement.

The electrical resistance of the mesh, array or fabric of electrically conductive filaments of the filament arrangement is preferably between 0.3 Ohms and 4 Ohms. More preferably, the electrical resistance of the mesh, array or fabric of electrically conductive filaments is between 0.5 Ohms and 3 Ohms, and more preferably about 1 Ohm. The electrical resistance of the mesh, array or fabric of electrically conductive filaments is preferably at least an order of magnitude, and more preferably at least two orders of magnitude, greater than the electrical resistance of the contact portions. This ensures that the heat generated by passing current through the filament arrangement is localised to the mesh or array of electrically conductive filaments. It is advantageous to have a low overall resistance for the filament arrangement if the system is powered by a battery. A low resistance, high current system allows for the delivery of high power to the filament arrangement. This allows the filament arrangement to heat the electrically conductive filaments to a desired temperature quickly.

The first and second electrically conductive contact portions may be fixed directly to the electrically conductive filaments. The contact portions may be positioned between the electrically conductive filaments and the electrically insulating substrate. For example, the contact portions may be formed from a copper foil that is plated onto the insulating substrate. The contact portions may also bond more readily with the filaments than the insulating substrate would.

In some embodiments a first electrically conductive contact portion may be located at an interior boundary line of the filament arrangement to the filament opening. The first electrically conductive contact portion may be guided through the capillary medium opening. A second electrically conductive contact portion may be located at an exterior boundary line of the filament arrangement.

Alternatively or additionally, the first and second electrically conductive contact portions may be integral with the electrically conductive filaments. For example, the filament arrangement may be formed by etching a conductive sheet to provide a plurality of filaments between two contact portions.

The housing of the liquid storage portion contains a capillary medium. A capillary medium is a material that actively conveys liquid from one end of the material to another. The capillary medium is advantageously oriented in the housing to convey liquid to the heater assembly.

The capillary medium may have a fibrous or spongy structure. The capillary medium preferably comprises a bundle of capillaries. For example, the capillary medium may comprise a plurality of fibres or threads or other fine bore tubes. The fibres or threads may be generally aligned to convey liquid to the heater. Alternatively, the capillary medium may comprise sponge-like or foam-like material. The structure of the capillary medium forms a plurality of small bores or tubes, through which the liquid can be transported by capillary action. The capillary medium may comprise any suitable material or combination of materials. Examples of suitable materials are a sponge or foam material, ceramic- or graphite-based materials in the form of fibres or sintered powders, foamed metal or plastics material, a fibrous material, for example made of spun or extruded fibres, such as cellulose acetate, polyester, or bonded polyolefin, polyethylene, terylene or polypropylene fibres, nylon fibres or ceramic. The capillary medium may have any suitable capillarity and porosity so as to be used with different liquid physical properties. The liquid has physical properties, including but not limited to viscosity, surface tension, density, thermal conductivity, boiling point and vapour pressure, which allow the liquid to be transported through the capillary device by capillary action.

The capillary medium is in contact with the electrically conductive filaments. The capillary medium may extend into interstices between the filaments. The heater assembly may draw liquid aerosol-forming substrate into the interstices by capillary action. The capillary medium may be in contact with the electrically conductive filaments over substantially the entire extent of the aperture. In one embodiment the capillary medium in contact with the electrically conductive filament arrangement may be a filamentary wick.

Advantageously, the heater assembly and the capillary medium may be sized to have approximately the same area. As used here, approximately means between that the heater assembly may be between 0-15 percent larger than the capillary medium. The shape of the heater assembly may also be similar to the shape of the capillary medium such that the assembly and the material substantially overlap. When the assembly and the material are substantially similar in size and shape, manufacturing can be simplified and the robustness of the manufacturing process improved. As discussed below, the capillary medium may include two or more capillary mediums including one or more layers of the capillary medium directly in contact with the mesh, array or fabric of electrically conductive filaments of the heater assembly in order to promote aerosol generation. The capillary mediums may include materials described herein.

At least one of the capillary mediums may be of sufficient volume in order to ensure that a minimal amount of liquid is present in said capillary medium to prevent "dry heating", which occurs if insufficient liquid is provided to the capillary medium in contact with the mesh, array or fabric of electrically conductive filaments. A minimum volume of said capillary medium may be provided in order to allow for between 20-40 puffs by the user. An average volume of liquid volatilized during a puff of a length between 1-4 seconds is typically between 1-4 milligrams of liquid. Thus, providing at least one capillary medium having a volume to retain between 20-160 milligrams of the liquid comprising the liquid-forming substrate may prevent the dry heating.

The housing may contain two or more different materials as capillary medium, wherein a first capillary medium, in contact with the filament arrangement, has a higher thermal decomposition temperature and a second capillary medium, in contact with the first capillary medium but not in contact with the filament arrangement has a lower thermal decomposition temperature. The first capillary medium effectively acts as a spacer separating the filament arrangement from the second capillary medium so that the second capillary medium is not exposed to temperatures above its thermal decomposition temperature. As used herein, "thermal decomposition temperature" means the temperature at which a material begins to decompose and lose mass by generation of gaseous by products. The second capillary medium may advantageously occupy a greater volume than the first capillary medium and may hold more aerosol-forming substrate that the first capillary medium. The second capillary medium may have superior wicking performance to the first capillary medium. The second capillary medium may be cheaper than the first capillary medium. The second capillary medium may be polypropylene.

The first capillary medium may separate the heater assembly from the second capillary medium by a distance of at least 1.5 millimeter, and preferably between 1.5 millimeter and 2 millimeter in order to provide a sufficient temperature drop across the first capillary medium.

The size and position of the capillary medium opening can be selected based on the airflow characteristics of the aerosol-generating system, or on the temperature profile of the heater assembly, or both. Position and shape of the capillary medium opening are arranged such that an airflow guided to the air impingement surface of the filament arrangement is whirled around the air impingement surface. In some embodiments, the capillary medium opening may be positioned towards the center of the cross section of the capillary medium. Preferably, the capillary medium opening is positioned in the center of the cross section of the capillary medium. Preferably, the capillary medium is of cylindrical shape. Preferably, the air duct through the capillary medium opening is of cylindrical shape.

The term "towards the center of the cross section of capillary medium" refers to a center portion of the cross section of the capillary medium that is away from the periphery of the capillary medium and has an area which is less than the total area of the cross section of the capillary medium. For example, the center portion may have an area of less than about 80 percent, less than about 60 percent, less than about 40 percent, or less than about 20 percent of the total area of the cross section of the capillary medium.

The filament opening may be positioned in a center portion of the filament arrangement, wherein the filament opening is extended by the capillary medium opening to form an air duct through the capillary medium. In this case, more aerosol passes through the filament arrangement in the center of the filament arrangement. This is advantageous in aerosol-generating systems in which the center of the filament arrangement is the most important vaporization area, for example in aerosol-generating systems in which the temperature of the heater assembly is higher in the center of the filament arrangement. Position and shape of the filament arrangement, of the filament opening, and of the capillary medium opening are arranged such that an airflow guided to the air impingement surface of the filament arrangement is whirled around the air impingement surface.

As used herein, the term "center portion" of the filament arrangement refers to a part of the filament arrangement that is away from the periphery of the filament arrangement and has an area which is less than the total area of the filament arrangement. For example, the center portion may have an area of less than about 80 percent, less than about 60 percent, less than about 40 percent, or less than about 20 percent of the total area of the filament arrangement.

An air inlet of the aerosol-generating system is arranged in the sidewalls of the main housing of the system. Ambient air is directed into the system and is guided to the air impingement surface of the heating assembly. The air stream arriving at the air impingement surface of the heater assembly is guided through the air duct defined by the capillary medium opening. The airflow entrains aerosols caused by heating the aerosol-forming substrate on the surface of the heater assembly. The aerosol containing air may then be guided along the cartridge between a cartridge housing and a main housing to the downstream end of the system, where it is mixed with ambient air from the further flow route (either before or upon reaching the downstream end). Guiding the aerosol through the air duct accelerates the airflow, thereby improving aerosolization through faster cooling.

The air inlets are provided in the sidewalls of the housing of the system, such that ambient air is drawn towards the heating element at an angle of approximately or up to 90° with respect to the air duct defined by the capillary medium opening. At least a large part of airflow is guided substantially parallel along the air impingement surface of the heater assembly and is then redirected into the air duct defined by the capillary medium. By the specific air flow routing of the present invention turbulences and vortices are created in the airflow, which efficiently carries the aerosol vapours. Further, the cooling rate may be increased which may also enhance aerosol formation.

The ambient air may also be guided through the air duct to the surface of the heater assembly, i.e. the direction of airflow is inverted as compared to the preferred direction of airflow. Also in this embodiment, guiding the ambient air through the air duct accelerates the airflow, thereby improving aerosolization.

An inlet opening of the second channel arranged in a region of a distal end of a cartridge housing may also be provided in an alternative system where a heating element is arranged at a proximal end of the cartridge. The second flow route may not only pass outside of the cartridge but also through the cartridge. Ambient air then enters the cartridge at a semi-open wall of the cartridge, passes through the cartridge and leaves the cartridge by passing though the heating element arranged at the proximal end of the cartridge. Thereby, ambient air may pass through the aerosol-forming substrate or through one or several channels arranged in a solid aerosol-forming substrate such that ambient air does not pass through the substrate itself but in the channels next to the substrate.

For allowing ambient air to enter a cartridge, a wall of the cartridge housing, preferably a wall opposite the heating element, preferably a bottom wall, is provided with at least one semi-open inlet. The semi-open inlet allows air to enter the cartridge but no air or liquid to leave the cartridge through the semi-open inlet. A semi-open inlet may for example be a semipermeable membrane, permeable in one direction only for air but is air- and liquid-tight in the opposite direction. A semi-open inlet may for example also be a one-way valve. Preferably the semi-open inlets allow air to pass through the inlet only if specific conditions are met, for example a minimum depression in the cartridge or a volume of air passing through the valve or membrane.

Such one-way valves may, for example, be commercially available valves, such as for example used in medical devices, for example LMS Mediflow One-Way, LMS SureFlow One-Way or LMS Check Valves (crosses membranes). Suitable membranes to be used for a cartridge having an airflow passing through the cartridge, are for example vented membranes as used in medical devices, for example Qosina Ref. 11066, vented cap with hydrophobic filter or valves as used in baby bottles. Such valves and membranes may be made of any material suitable for applications in electrically heated smoking systems. Materials suitable for medical devices and FDA approved materials may be used; for example Graphene having very high mechanical resistance and thermal stability within a large range of temperatures. Preferably, valves are made of soft resilient material for supporting a liquid-tight incorporation of the one or several valves into a wall of the container housing.

Letting ambient air pass through the substrate supports an aerosolization of the aerosol-forming substrate. During puffing, a depression occurs in the cartridge, which may activate the semi-open inlets. Ambient air then passes the cartridge, preferably a high retention or high release material (HRM) or a liquid, and crosses the heating element, thereby creating and sustaining aerosolization of the liquid, when the heating element sufficiently heats the liquid. In addition, due to the depression caused during puffing, a supply of liquid in a transport material such as a capillary medium to the heating element may be limited. An ambient airflow through the cartridge may equalize pressure differences within the cartridge and thereby support an unhindered capillary action towards the heating element.

A semi-open inlet may, in addition, or alternatively also be provided in one or several side walls of the cartridge housing. Semi-open inlets in side walls provide a lateral airflow into the cartridge towards the open top end of the cartridge housing, where the heating element is arranged. Preferably, lateral airflows pass through the aerosol-forming substrate.

The system may further comprise electric circuitry connected to the heater assembly and to an electrical power source, the electric circuitry configured to monitor the electrical resistance of the heater assembly or of one or more filaments of the heater assembly, and to control the supply of power to the heater assembly dependent on the electrical resistance of the heater assembly or the one or more filaments.

The electric circuitry may comprise a microprocessor, which may be a programmable microprocessor. The electric circuitry may comprise further electronic components. The electric circuitry may be configured to regulate a supply of power to the heater assembly. Power may be supplied to the heater assembly continuously following activation of the system or may be supplied intermittently, such as on a puff-by-puff basis. The power may be supplied to the heater assembly in the form of pulses of electrical current.

The system advantageously comprises a power supply, typically a battery, within the main body of the housing. As an alternative, the power supply may be another form of charge storage device such as a capacitor. The power supply may require recharging and may have a capacity that allows for the storage of enough energy for one or more smoking experiences; for example, the power supply may have sufficient capacity to allow for the continuous generation of aerosol for a period of around six minutes or for a period that is a multiple of six minutes. In another example, the power supply may have sufficient capacity to allow for a predetermined number of puffs or discrete activations of the heater assembly.

Preferably, the aerosol generating system comprises a housing. Preferably, the housing is elongate. The housing may comprise any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK) and polyethylene. Preferably, the material is light and non-brittle.

The aerosol-forming substrate is a substrate capable of releasing volatile compounds that can form an aerosol. The volatile compounds may be released by heating the aerosol-forming substrate. The aerosol-forming substrate may comprise plant-based material. The aerosol- forming substrate may comprise tobacco. The aerosol-forming substrate may comprise a tobacco-containing material containing volatile tobacco flavour compounds, which are released from the aerosol-forming substrate upon heating. The aerosol-forming substrate may alternatively comprise a non-tobacco-containing material. The aerosol-forming substrate may comprise homogenised plant-based material. The aerosol-forming substrate may comprise homogenised tobacco material. The aerosol-forming substrate may comprise at least one aerosol-former. The aerosol-forming substrate may comprise other additives and ingredients, such as flavourants.

The aerosol-generating system may comprise a main unit and a cartridge that is removably coupled to the main unit, wherein the liquid storage portion and heater assembly are provided in the cartridge and the main unit comprises a power supply.

The aerosol-generating system may be an electrically operated smoking system. Preferably, the aerosol-generating system is portable. The aerosol-generating system may have a size comparable to a conventional cigar or cigarette. The smoking system may have a total length between approximately 30 millimeter and approximately 150 millimeter. The smoking system may have an external diameter between approximately 5 millimeter and approximately 30 millimeter.

In the method of manufacture of a cartridge for use in an electrically operated aerosol-generating system, the step of filling the liquid storage portion may be performed before or after the step of providing the heater assembly. The heater assembly may be fixed to the housing of the liquid storage portion. The step of fixing may, for example, comprise heat sealing, gluing or welding the heater assembly to the housing of the liquid storage portion.

Features described in relation to one aspect may equally be applied to other aspects of the invention.

As used herein, "electrically conductive" means formed from a material having a resistivity of 1 x 10⁻⁴ Ohm meters, or less.

As used herein, "electrically insulating" means formed from a material having a resistivity of 1x10⁴ Ohm meters or more.

As used herein "fluid permeable" in relation to a heater assembly means that the aerosol-forming substrate, in a gaseous phase and possibly in a liquid phase, can readily pass through the heater assembly.

Embodiments of the invention will now be described, by way of example only, with reference to the accompanying drawings, in which:
Figure 1 is a perspective topside view of an arrangement comprising a heater assembly and a capillary medium, in accordance with an embodiment of the invention;
Figure 2A is a perspective topside view of a heater assembly comprising a filament arrangement of planar shape with a central opening;
Figure 2B is a perspective topside view of a heater assembly comprising a filament arrangement of curved shape with a central opening;
Figure 2C is a perspective topside view of a heater assembly comprising a filament arrangement of funnel shape with a central opening;
Figure 3 is a perspective topside view of a capillary medium comprising a first capillary medium and a second capillary medium with both having a central opening;
Figure 4A is a perspective topside view of an arrangement comprising a heater assembly and a capillary medium, in accordance with an embodiment of the invention;
Figure 4B is a perspective topside view of an arrangement comprising a heater assembly and a capillary medium, in accordance with an embodiment of the invention; and
Figure 5 is a schematic illustration of a system, incorporating a cartridge comprising a heater assembly and a capillary medium, in accordance with an embodiment of the invention.

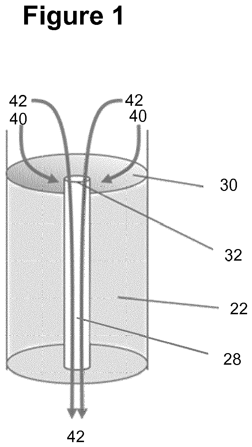

Figure 1 shows a filament arrangement 30 according to one of the embodiments of the present disclosure. The filament arrangement has a filament opening 32. A capillary medium 22 is in contact with the filament arrangement 30. The capillary medium has a capillary medium opening 28 that acts as an air duct through the capillary medium 22. Ambient air is guided in airflow 40 to the air impingement surface of the filament arrangement 30. The suction of the air duct through the capillary medium 22 causes an acceleration of the airflow so that the volatized vapors are drawn in an airflow 42 through the air duct.

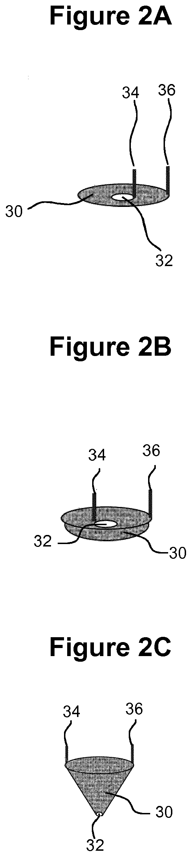

Figures 2A to 2C illustrate various shapes of filament arrangements 30, each having a filament opening 32 in a center portion of the filament arrangement 30.

Figure 2A shows a planar filament arrangement 30. Turbulences and vortexes of the airflow 40 on the air impingement surface of the filament arrangement are caused by the central opening acting as an entrance to the air duct of the capillary medium opening 28.

Figure 2B shows a non-planar filament arrangement 30 that is curved along one dimension. The curved shape causes a whirling of the airflow 40 on the air impingement surface. This effect is further increased by the optional filament opening 32.

Figure 2C shows a non-planar filament arrangement 30 having a funnel shape with an optional filament opening 32 at the bottom of the funnel shaped filament arrangement 30. The funnel shape causes a whirling of the airflow 40 on the air impingement surface. This effect is further increased by the optional filament opening 32.

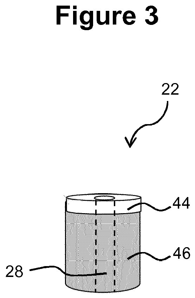

Figure 3 shows a capillary medium 22 to be used in an aerosol-generating system. There are two separate capillary mediums 44, 46 in use. A larger body of a second capillary medium 46 is provided on an opposite side of the first capillary medium 44 that is in contact with the filament arrangement 30 of the heater assembly. Both the first capillary medium 44 and the second capillary medium 46 retain liquid aerosol-forming substrate. The first capillary medium 44, which contacts the filament arrangement, has a higher thermal decomposition temperature (at least 160 degrees Celsius or higher such as approximately 250 degrees Celsius) than the second capillary medium 46. The first capillary medium 44 effectively acts as a spacer separating the filament arrangement 30 from the second capillary medium 46 so that the second capillary medium is not exposed to temperatures above its thermal decomposition temperature. The first capillary medium 44 is flexible and preferably accommodates to the non-planar shape of the heater assembly, such that the contact surface between the capillary medium and the heater assembly is maximized.

The thermal gradient across the first capillary medium is such that the second capillary medium is exposed to temperatures below its thermal decomposition temperature. The second capillary medium 46 may be chosen to have superior wicking performance to the first capillary medium 44, may retain more liquid per unit volume than the first capillary medium and may be less expensive than the first capillary medium. The capillary medium 22 comprises a capillary medium opening 28 acting as an air duct through the capillary medium 22.

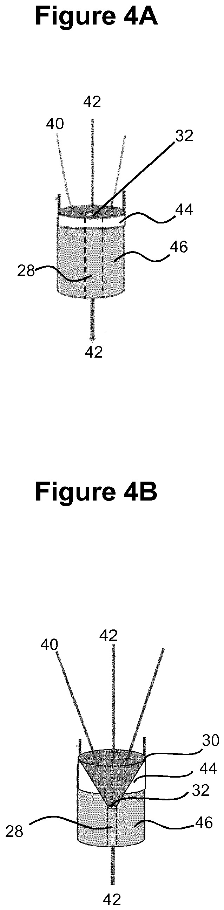

Figures 4A and 4B illustrate the inventive combination of a filament arrangement 30 with two separate capillary mediums 44, 46 that guides the airflow 42 through an air duct defined by the capillary medium opening 28 after being mixed with volatized vapors on the surface of the filament arrangement 30. Alternatively, the airflow may be guided in the reverse direction, i.e. the ambient air may be guided as airflow 40 through the air duct to the surface of the filament arrangement 30.

Figure 4A shows a planar filament arrangement 30 with a filament opening 32 that extends the capillary medium opening 28. The air duct through the capillary mediums 44, 46 accelerates the airflow and improves aerosolization.

Figure 4B shows a non-planar filament arrangement 30 of a funnel shape with a filament opening 32 at the bottom end of the filament arrangement 30, the filament opening 32 extending the capillary medium opening 28. The funnel shape creates turbulences and vortexes that encourage the mixing of the volatized vapors with the ambient air.

In the embodiment depicted in Figure 4B the lower portion of the filament arrangement 30 is in direct contact with the second capillary medium 46. Of course the size of capillary medium 44 can also be increased, such that it covers the complete filament arrangement 30, and such that direct contact between the filament arrangement 30 and the second capillary medium 46 is prevented.

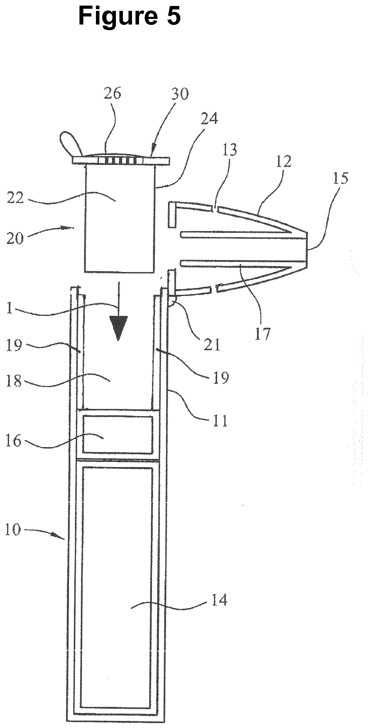

Figure 5 is a schematic illustration of an aerosol-generating system, including a cartridge 20 with a heater assembly comprising a filament arrangement 30 according to one of the embodiments of the present disclosure and with a capillary medium 22 according to one of the embodiments of the present disclosure. The aerosol-generating system comprises an aerosol-generating device 10 and a separate cartridge 20. In this example, the aerosol-generating system is an electrically operated smoking system.

The cartridge 20 contains an aerosol-forming substrate and is configured to be received in a cavity 18 within the device. Cartridge 20 should be replaceable by a user when the aerosol-forming substrate provided in the cartridge 20 is depleted. Figure 5 shows the cartridge 20 just prior to insertion into the device, with the arrow 1 in Figure 5 indicating the direction of insertion of the cartridge 20. The heater assembly with the filament arrangement 30 and the capillary medium 22 is located in the cartridge 20 behind a cover 26. The aerosol-generating device 10 is portable and has a size comparable to a conventional cigar or cigarette. The device 10 comprises a main body 11 and a mouthpiece portion 12. The main body 11 contains a power supply 14, for example a battery such as a lithium iron phosphate battery, control electronics 16 and a cavity 18. The mouthpiece portion 12 is connected to the main body 11 by a hinged connection 21 and can move between an open position as shown in Figure 5 and a closed position. The mouthpiece portion 12 is placed in the open position to allow for insertion and removal of cartridges 20 and is placed in the closed position when the system is to be used to generate aerosol. The mouthpiece portion comprises a plurality of air inlets 13 and an outlet 15. In use, a user sucks or puffs on the outlet to draw air from the air inlets 13, through the mouthpiece portion and the cartridge 20 to the outlet 15, and thereafter into the mouth or lungs of the user. Internal baffles 17 are provided to force the air flowing through the mouthpiece portion 12 past the cartridge.

The cavity 18 has a circular cross-section and is sized to receive a housing 24 of the cartridge 20. Electrical connectors 19 are provided at the sides of the cavity 18 to provide an electrical connection between the control electronics 16 and battery 14 and corresponding electrical contacts on the cartridge 20.

Other cartridge designs incorporating a heater assembly with a filament arrangement 30 in accordance with this disclosure and a capillary medium 22 in accordance with this disclosure can now be conceived by one of ordinary skill in the art. For example, the cartridge 20 may include a mouthpiece portion 12, may include more than one heater assembly and may have any desired shape. Furthermore, a heater assembly in accordance with the disclosure may be used in systems of other types to those already described, such as humidifiers, air fresheners, and other aerosol-generating systems.

The exemplary embodiments described above illustrate but are not limiting. In view of the above discussed exemplary embodiments, other embodiments consistent with the above exemplary embodiments will now be apparent to one of ordinary skill in the art.

## Claims

1. An aerosol-generating system comprising:
a liquid storage portion comprising a housing holding a liquid aerosol-forming substrate and a capillary medium (22), the housing having an opening;
a fluid permeable heater assembly comprising an arrangement (30) of electrically conductive filaments arranged to define an air impingement surface, wherein the fluid permeable heater assembly extends across the opening of the housing, and wherein the filament arrangement (30) defines a filament opening (32) allowing airflow (42) to pass through;
wherein the capillary medium (22) is provided in contact with the heater assembly;
wherein the liquid aerosol-forming substrate is drawn via the capillary medium (22) to the electrically conductive filament arrangement (30); and
wherein the capillary medium (22) comprises a capillary medium opening (28) extending the filament opening (32) through the capillary medium (22), and
wherein the aerosolgenerating system comprises a main housing and an air inlet provided in a sidewall of the main housing, such that ambient air is drawn towards the heating element from the air inlet at an angle of up to 90° with respect to an air duct defined by the capillary medium opening (28).

2. An aerosol-generating system according to claim 1, wherein the filament arrangement (30) is of planar shape.

3. An aerosol-generating system according to claim 1, wherein the filament arrangement (30) is of non-planar shape.

4. An aerosol-generating system according to claim 3, wherein the filament arrangement (30) is curved along one or more dimensions.

5. An aerosol-generating system according to claims 3 or 4, wherein the filament arrangement (30) is funnel shaped.

6. An aerosol-generating system according to any preceding claim, wherein the capillary medium (22) is of cylindrical shape, and wherein the capillary medium opening (28) is a central opening.

7. An aerosol-generating system according to any preceding claim, wherein the aerosol-generating system is configured such that the liquid vaporized at the fluid permeable heater assembly (30) is transported by an airflow (42) through the capillary medium opening (28), and wherein guiding the airflow (42) through the capillary medium opening (28) causes accelerating the airflow (42).

8. An aerosol-generating system according to any preceding claim, wherein position and shape of the filament arrangement (30) are dimensioned and arranged such that an airflow (40) guided to the air impingement surface of the filament arrangement (30) is whirled around the air impingement surface.

9. An aerosol-generating system according to any preceding claim, wherein the heater assembly comprises a first electrically conductive contact portion (34) located at an interior boundary line of the filament arrangement (30) to the filament opening (32) and a second electrically conductive contact portion (36) located at an exterior boundary line of the filament arrangement (30), and wherein the first electrically conductive contact portion (34) is guided through the capillary medium opening (28).

10. An aerosol-generating system according to any preceding claim, wherein the system comprises a main unit (10) and a cartridge (20) that is removably coupled to the main unit (10), wherein the liquid storage portion and heater assembly are provided in the cartridge (20) and the main unit (10) comprises a power supply (14).

11. An aerosol-generating system according to any preceding claim, wherein the system is an electrically operated smoking system.

12. A method of manufacture of a cartridge for use in an electrically operated aerosol-generating system, comprising:
providing a liquid storage portion comprising a housing having an opening;
providing a capillary material (22) within the liquid storage portion;
filling the liquid storage portion with liquid aerosol-forming substrate; and
providing a fluid permeable heater assembly comprising an arrangement (30) of electrically conductive filaments arranged to define an air impingement surface, wherein the fluid permeable heater assembly extends across the opening of the housing, and wherein the filament arrangement (30) defines a filament opening (32) allowing airflow (42) to pass through;
wherein the capillary medium (22) is provided in contact with the heater assembly; and
wherein the capillary medium (22) comprises a capillary medium opening (28) allowing airflow (42) to pass through the capillary medium (22), and
providing a main housing and an air inlet in a sidewall of the main housing, such that ambient air is drawn towards the heating element from the air inlet at an angle of approximately or up to 90° with respect to an air duct defined by the capillary medium opening (28).

13. The method of claim 12, wherein the fluid permeable heater assembly is formed from an initially flat filament arrangement (30) that is deformed to define a non-planar air impingement surface.

14. The method of claims 12 or 13, wherein the fluid permeable heater assembly is fixed to the housing of the liquid storage portion by heat sealing, gluing or welding the heater assembly to the housing of the liquid storage portion.

## Patentansprüche

1. Aerosolerzeugungssystem, aufweisend:
einen Flüssigspeicherteil, der ein Gehäuse aufweist, das ein flüssiges aerosolbildendes Substrat und ein Kapillarmedium (22) enthält, wobei das Gehäuse eine Öffnung aufweist;
eine fluiddurchlässige Heizvorrichtungsbaugruppe, die eine Anordnung (30) von elektrisch leitenden Fäden aufweist, die ausgeführt sind, eine Luftaufprallfläche zu definieren, wobei sich die fluiddurchlässige Heizvorrichtungsbaugruppe über die Öffnung des Gehäuses hinweg erstreckt, und wobei die Fadenanordnung (30) eine Fadenöffnung (32) definiert, die ermöglicht, dass ein Luftstrom (42) hindurchströmt;
wobei das Kapillarmedium (22) in Kontakt mit der Heizvorrichtungsbaugruppe vorgesehen ist;
wobei das flüssige aerosolbildende Substrat über das Kapillarmedium (22) zu der elektrisch leitenden Fadenanordnung (30) gezogen wird; und
wobei das Kapillarmedium (22) eine Kapillarmediumöffnung (28) aufweist, welche die die Fadenöffnung (32) durch das Kapillarmedium (22) erstreckt, und
wobei das Aerosolerzeugungssystem ein Hauptgehäuse und einen Lufteinlass aufweist, der in einer Seitenwand des Hauptgehäuses vorgesehen ist, sodass Umgebungsluft von dem Lufteinlass zu dem Heizelement in einem Winkel von bis zu 90° in Bezug auf einen Luftkanal, der durch die Kapillarmediumöffnung (28) definiert ist, gezogen wird.

2. Aerosolerzeugungssystem nach Anspruch 1, wobei die Fadenanordnung (30) von planarer Form ist.

3. Aerosolerzeugungssystem nach Anspruch 1, wobei die Fadenanordnung (30) von nichtplanarer Form ist.

4. Aerosolerzeugungssystem nach Anspruch 3, wobei die Fadenanordnung (30) entlang einer oder mehrerer Abmessungen gekrümmt ist.

5. Aerosolerzeugungssystem nach den Ansprüchen 3 oder 4, wobei die Fadenanordnung (30) trichterförmig ist.

6. Aerosolerzeugungssystem nach einem der vorstehenden Ansprüche, wobei das Kapillarmedium (22) von zylindrischer Form ist, und wobei die Kapillarmediumöffnung (28) eine zentrale Öffnung ist.

7. Aerosolerzeugungssystem nach einem der vorstehenden Ansprüche, wobei das Aerosolerzeugungssystem derart konfiguriert ist, dass die Flüssigkeit, die an der fluiddurchlässigen Heizvorrichtungsbaugruppe (30) verdampft wird, mittels eines Luftstroms (42) durch die Kapillarmediumöffnung (28) transportiert wird, und wobei das Lenken des Luftstroms (42) durch die Kapillarmediumöffnung (28) das Beschleunigen des Luftstroms (42) bewirkt.

8. Aerosolerzeugungssystem nach einem der vorstehenden Ansprüche, wobei Position und Form der Fadenanordnung (30) derart dimensioniert und angeordnet sind, dass ein Luftstrom (40), der zu der Luftaufprallfläche der Fadenanordnung (30) gelenkt wird, um die Luftaufprallfläche herumgewirbelt wird.

9. Aerosolerzeugungssystem nach einem der vorstehenden Ansprüche, wobei die Heizvorrichtungsbaugruppe einen ersten elektrisch leitenden Kontaktabschnitt (34) aufweist, der an einer inneren Grenzlinie der Fadenanordnung (30) zur Fadenöffnung (32) angeordnet ist, und einen zweiten elektrisch leitenden Kontaktabschnitt (36), der an einer äußeren Grenzlinie der Fadenanordnung (30) angeordnet ist, und wobei der erste elektrisch leitende Kontaktabschnitt (34) durch die Kapillarmediumöffnung (28) gelenkt wird.

10. Aerosolerzeugungssystem nach einem der vorstehenden Ansprüche, wobei das System eine Haupteinheit (10) und eine Patrone (20) aufweist, die mit der Haupteinheit (10) lösbar gekoppelt ist, wobei der Flüssigspeicherteil und die Heizvorrichtungsbaugruppe in der Patrone (20) vorgesehen sind und die Haupteinheit (10) eine Stromversorgung (14) aufweist.

11. Aerosolerzeugungssystem nach einem der vorstehenden Ansprüche, wobei das System ein elektrisch betriebenes System zum Rauchen ist.

12. Verfahren zum Herstellen einer Patrone zum Gebrauch in einem elektrisch betriebenen Aerosolerzeugungssystem, aufweisend:
Vorsehen eines Flüssigspeicherteils mit einem Gehäuse mit einer Öffnung;
Vorsehen eines Kapillarmaterials (22) innerhalb des Flüssigspeicherteils;
Füllen des Flüssigspeicherteils mit flüssigem aerosolbildendem Substrat; und
Vorsehen einer fluiddurchlässigen Heizvorrichtungsbaugruppe, die eine Anordnung (30) von elektrisch leitenden Fäden aufweist, die ausgeführt sind, eine Luftaufprallfläche zu definieren, wobei sich die fluiddurchlässige Heizvorrichtungsbaugruppe über die Öffnung des Gehäuses hinweg erstreckt, und wobei die Fadenanordnung (30) eine Fadenöffnung (32) definiert, die ermöglicht, dass ein Luftstrom (42) hindurchströmt;
wobei das Kapillarmedium (22) in Kontakt mit der Heizvorrichtungsbaugruppe vorgesehen ist; und
wobei das Kapillarmedium (22) eine Kapillarmediumöffnung (28) umfasst, die ermöglicht, dass ein Luftstrom (42) durch das Kapillarmedium (22) hindurchströmt, und
Vorsehen eines Hauptgehäuses und eines Lufteinlasses in einer Seitenwand des Hauptgehäuses, sodass Umgebungsluft von dem Lufteinlass zu dem Heizelement in einem Winkel von ca. oder bis zu 90° in Bezug auf einen Luftkanal, der durch die Kapillarmediumöffnung (28) definiert ist, gezogen wird.

13. Verfahren nach Anspruch 12, wobei die fluiddurchlässige Heizvorrichtungsbaugruppe aus einer anfänglich flachen Fadenanordnung (30) gebildet wird, die deformiert wird, um eine nichtplanare Luftaufprallfläche zu definieren.

14. Verfahren nach den Ansprüchen 12 oder 13, wobei die fluiddurchlässige Heizvorrichtungsbaugruppe durch Heißversiegeln, Kleben oder Schweißen der Heizvorrichtungsbaugruppe an das Gehäuse des Flüssigspeicherteils am Gehäuse des Flüssigspeicherteils befestigt wird.

## Revendications

1. Système de génération d'aérosol, comprenant :
une partie de stockage de liquide comprenant un logement contenant un substrat formant aérosol liquide et un milieu capillaire (22), le logement ayant une ouverture ;
un ensemble de chauffage perméable aux liquides comprenant un agencement (30) de filaments électriquement conducteurs disposés pour définir une surface d'impact de l'air, où l'ensemble de chauffage perméable aux liquides s'étend sur l'ouverture du logement, et où l'agencement de filaments (30) définit une ouverture du filament (32) laissant passer le flux d'air (42) ;
dans lequel le milieu capillaire (22) est fourni en contact avec l'ensemble de chauffage ;
dans lequel le substrat formant aérosol liquide est aspiré via le milieu capillaire (22) vers l'agencement de filaments électriquement conducteurs (30) ; et
dans lequel le milieu capillaire (22) comprend une ouverture du milieu capillaire (28) étendant l'ouverture du filament (32) à travers le milieu capillaire (22), et
dans lequel le système de génération d'aérosol comprend un logement principal et une entrée d'air fournie dans une paroi latérale du logement principal, de telle sorte que l'air ambiant est aspiré vers l'élément de chauffage de l'entrée d'air à un angle allant jusqu'à 90° par rapport à un conduit d'air défini par l'ouverture du milieu capillaire (28).

2. Système de génération d'aérosol selon la revendication 1, dans lequel l'agencement de filaments (30) est de forme planaire.

3. Système de génération d'aérosol selon la revendication 1, dans lequel l'agencement de filaments (30) est de forme non planaire.

4. Système de génération d'aérosol selon la revendication 3, dans lequel l'agencement de filaments (30) est courbé le long d'une ou de plusieurs dimensions.

5. Système de génération d'aérosol selon les revendications 3 ou 4, dans lequel l'agencement de filaments (30) est en forme d'entonnoir.

6. Système de génération d'aérosol selon l'une quelconque des revendications précédentes, dans lequel le milieu capillaire (22) est de forme cylindrique, et dans lequel l'ouverture moyenne capillaire (28) est une ouverture centrale.

7. Système de génération d'aérosol selon l'une quelconque des revendications précédentes, dans lequel le système de génération d'aérosol est configuré de telle sorte que le liquide vaporisé au niveau de l'ensemble de chauffage perméable aux liquides (30) est transporté par un flux d'air (42) à travers l'ouverture du milieu capillaire (28), et dans lequel le guidage du flux d'air (42) par l'ouverture du milieu capillaire (28) provoque l'accélération du flux d'air (42).

8. Système de génération d'aérosol selon l'une quelconque des revendications précédentes, dans lequel la position et la forme de l'agencement de filaments (30) sont dimensionnées et agencées de telle sorte qu'un flux d'air (40) guidé vers la surface d'impact de l'air de l'agencement de filaments (30) est dévié autour de la surface d'impact de l'air.

9. Système de génération d'aérosol selon l'une quelconque des revendications précédentes, dans lequel l'ensemble de chauffage comprend une première partie de contact électriquement conductrice (34) située à une ligne de délimitation intérieure de l'agencement de filaments (30) vers l'ouverture du filament (32) et une deuxième partie de contact électriquement conductrice (36) située à une ligne de délimitation extérieure de l'agencement de filaments (30), et dans lequel la première partie de contact électriquement conductrice (34) est guidée à travers l'ouverture du milieu capillaire (28).

10. Système de génération d'aérosol selon l'une quelconque des revendications précédentes, dans lequel le système comprend une unité principale (10) et une cartouche (20) qui est couplée de manière amovible à l'unité principale (10), dans lequel la partie de stockage de liquide et l'ensemble de chauffage sont fournis dans la cartouche (20) et l'unité principale (10) comprend une alimentation électrique (14).

11. Système de génération d'aérosol selon l'une quelconque des revendications précédentes, dans lequel le système est un système à fumer à fonctionnement électrique.

12. Procédé de fabrication d'une cartouche destinée à être utilisée dans un système de génération d'aérosol à fonctionnement électrique, comprenant :
la fourniture d'une partie de stockage de liquide comprenant un logement ayant une ouverture ;
la fourniture d'un matériau capillaire (22) dans la partie de stockage de liquide ;
le remplissage de la partie de stockage de liquide avec un substrat formant aérosol liquide ; et
la fourniture d'un ensemble de chauffage perméable aux liquides comprenant un agencement (30) de filaments électriquement conducteurs disposés pour définir une surface d'impact de l'air, où l'ensemble de chauffage perméable aux liquides s'étend sur l'ouverture du logement, et où l'agencement de filaments (30) définit une ouverture du filament (32) laissant passer le flux d'air (42) ;
dans lequel le milieu capillaire (22) est fourni en contact avec l'ensemble de chauffage ; et
dans lequel le milieu capillaire (22) comprend une ouverture du milieu capillaire (28) permettant au flux d'air (42) de passer par le milieu capillaire (22), et
la fourniture d'un logement principal et d'une entrée d'air dans une paroi latérale du logement principal, de sorte que l'air ambiant soit aspiré vers l'élément de chauffage de l'entrée d'air à un angle d'environ ou jusqu'à 90° en ce qui concerne un conduit d'air défini par l'ouverture du milieu capillaire (28).

13. Procédé selon la revendication 12, dans lequel l'ensemble de chauffage perméable aux liquides est formé à partir d'un agencement de filaments initialement plat (30) qui est déformé pour définir une surface d'impact de l'air non planaire.

14. Procédé selon les revendications 12 ou 13, dans lequel l'ensemble de chauffage perméable aux liquides est fixé au logement de la partie de stockage de liquide par thermoscellage, collage ou soudure de l'ensemble de chauffage vers le logement de la partie de stockage de liquide.
